(19) Europäisches Patentamt
European Patent Office
Office européen des brevets

(11) EP 1 030 212 A2

(12) EUROPÄISCHE PATENTANMELDUNG

(43) Veröffentlichungstag:
**23.08.2000 Patentblatt 2000/34**

(51) Int. Cl.[7]: **G02F 1/15**, C07D 241/46

(21) Anmeldenummer: **00102139.3**

(22) Anmeldetag: **07.02.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **18.02.1999 DE 19906655**

(71) Anmelder:
**Bayer Aktiengesellschaft**
**51368 Leverkusen (DE)**

(72) Erfinder:
- **Berneth, Horst, Dr.**
  **51373 Leverkusen (DE)**
- **Neigl, Ralf, Dr.**
  **51373 Leverkusen (DE)**

(54) **Elektrochrome Vorrichtung mit verbesserter Lichtechtheit**

(57) Die Verwendung bestimmter Dihydronaphthazine oder Dihydrophenazine in elektrochromen Vorrichtungen führt zu einer verbesserten Lichtbeständigkeit der elektrochromen Vorrichtungen.

EP 1 030 212 A2

**Beschreibung**

Die vorliegende Erfindung betrifft eine elektrochrome Vorrichtung mit verbesserter Lichtechtheit.

Elektrochrome Vorrichtungen sind bereits bekannt, beispielsweise aus D. Theis in Ullmann's Encyclopedia of Industrial Chemistry, Vol. A 8, S. 622, Verlag Chemie 1987 und WO-A 94/23333. Man unterscheidet zwei Grundtypen:

<u>Typ 1:</u> vollflächige elektrochrome Vorrichtung.

<u>Typ 2:</u> elektrochrome Anzeigevorrichtungen mit strukturierten Elektroden.

Typ 1 findet beispielsweise bei elektrisch abdunkelbaren Fensterscheiben oder elektrisch abblendbaren Autospiegeln Anwendung. Solche Vorrichtungen sind beispielsweise aus US-A 4 902 108 bekannt.

Typ 2 findet bei Segment- und Matrixanzeigen Verwendung. Solche Anzeigevorrichtungen sind beispielsweise in DE-A 196 31 728 vorgeschlagen worden. Derartige Vorrichtungen können transmissiv oder bei Verspiegelung reflektiv betrachtet werden.

In WO-A 94/23333 werden elektrochrome Materialien verschiedener Bauweise gegenübergestellt, die aber nicht als Anzeigevorrichtungen verwendet werden:

<u>Bauweise a:</u> Die elektrochromen Substanzen liegen als Film oder Schicht fest auf den Elektroden (Ullmann, s.o.).

<u>Bauweise b:</u> Die elektrochromen Substanzen werden beim Redoxprozess auf den Elektroden als Schicht abgeschieden ( Ullmann, s.o.).

<u>Bauweise c</u>: Die elektrochromen Substanzen bleiben permanent in Lösung.

Für Bauweise a) ist als elektrochromes Material das Paar Wolframoxid/Palladiumhydrid das bekannteste.

Für Bauweise b) sind Viologene als elektrochrome Substanzen beschrieben worden. Diese Vorrichtungen sind nicht selbstlöschend, das erzeugte Bild bleibt also nach dem Abschalten des Stromes bestehen und kann nur durch Umpolen der Spannung wieder gelöscht werden. Solche Vorrichtungen sind nicht besonders beständig und erlauben keine hohe Zahl an Schaltzyklen.

Zudem sind insbesondere solche mit Wolframoxid/Palladiumhydrid aufgebauten Zellen wegen der Lichtstreuung an diesen elektrochromen Schichten nicht im durchfallenden Licht zu betreiben, sondern lediglich reflektiv.

Aus Elektrokhimiya **13,** 32-37 (1977), **13,** 404-408, **14,** 319-322 (1978), US-A 4 902 108 und US-A 5 140 455 ist ein elektrochromes System dieser letztgenannten Bauweise c) bekannt. In einer elektrochromen Zelle, die aus leitfähig beschichteten Glasplatten aufgebaut ist, ist eine Lösung eines Paares elektrochromer Substanzen in einem inerten Lösungsmittel enthalten.

Als Paar von elektrochromen Substanzen wird je eine elektrochemisch reversibel reduzierbare und eine reversibel oxidierbare Substanz verwendet. Beide sind im Grundzustand farblos oder nur schwach gefürbt. Unter Einfluss einer elektrischen Spannung wird die eine Substanz reduziert, die andere oxidiert, wobei beide farbig werden. Nach Abschalten der Spannung bildet sich bei beiden Substanzen der Grundzustand wieder zurück, wobei Entfärbung bzw. Farbaufhellung auftritt.

$$RED_1 + OX_2 \rightleftarrows OX_1 + RED_2$$

(farblos) (Niederenergiepaar) — (farbig) (Hochenergiepaar)

Aus US-A 4 902 108 ist bekannt, dass solche Paare von Redoxsubstanzen geeignet sind, bei denen die reduzierbare Substanz wenigstens zwei chemisch reversible Reduktionswellen im Cyclischen Voltammogramm und die oxidierbare Substanz entsprechend wenigstens zwei chemisch reversible Oxidationswellen besitzt.

Gemäß WO-A 94/23333 haben derartige Lösungssysteme der Bauweise c) jedoch gravierende Nachteile.

Die Diffusion der elektrochromen Substanzen in der Lösung bedingt unscharfe Farbgrenzen und verursacht einen hohen Stromverbrauch zur Aufrechterhaltung des gefärbten Zustandes, da die gefärbten Substanzen durch Rekombination und Reaktion an der jeweils gegenüberliegenden Elektrode permanent abgebaut werden.

Nichtsdestoweniger sind für solche elektrochromen Zellen der Bauweise c) verschiedene Anwendungen beschrieben worden. So können sie beispielsweise als Automobilrückspiegel ausgebildet sein, der bei Nachtfahrt durch Anlegen einer Spannung abgedunkelt werden kann und somit das Blenden durch Scheinwerfer nachfolgender Fahrzeuge verhindert ( US-A 3 280 701, US-A 4 902 108, EP-A 0 435 689). Weiterhin können solche Zellen auch in Fensterscheiben oder Auto-Sonnendächern eingesetzt werden, wo sie nach Anlegen einer Spannung das Sonnenlicht abdunkeln. Ebenfalls beschrieben ist die Anwendung solcher Vorrichtungen als elektrochrome Anzeigevorrichtungen, beispielsweise in Segment- oder Matrix-Displays mit strukturierten Elektroden ( DE-A 196 31 728).

Die elektrochromen Zellen bestehen normalerweise aus einem Paar Glasplatten, von denen im Falle des Autospiegels eine verspiegelt ist. Eine Seite dieser Scheiben ist mit einer lichtdurchlässigen, elektrisch leitfähigen Schicht, beispielsweise Indium-Zinn-Oxid (ITO), flächig beschichtet, wobei im Falle der Anzeigevorrichtungen diese leitfähige Beschichtung in elektrisch voneinander getrennte Segmente aufgeteilt ist, die einzeln kontaktiert sind. Aus diesen Scheiben wird nun eine Zelle aufgebaut, indem sie mit ihrer einander zugewandten elektrisch leitfähig beschichteten Seite über einen Dichtungsring zu einer Zelle verbunden werden. In diese Zelle wird nun über eine Öffnung eine elektrochrome Flüssigkeit eingefüllt und die Zelle dicht verschlossen. Über die ITO-Schichten werden die beiden Scheiben mit einer Spannungsquelle verbunden.

Die vorstehend beschriebenen elektrochromen Vorrichtungen zeigen in der Regel eine Empfindlichkeit gegenüber Licht, insbesondere UV-Licht. Es sind deshalb beispielsweise in US-A 5 280 380 elektrochrome Vorrichtungen beschrieben, die UV-Absorber enthalten.

Gegenüber der Verwendung von UV-Absorbern wäre die Verwendung von elektrochromen Verbindungen vorteilhaft, die inherent eine bessere Lichtbeständigkeit besitzen.

Überraschenderweise wurde nun gefunden, dass die Verwendung bestimmter Dihydronapthazine oder Dihydrophenazine zu einer verbesserten Lichtbeständigkeit der elektrochromen Vorrichtung führt.

Gegenstand der Erfindung ist demnach eine elektrochrome Vorrichtung, enthaltend ein Paar Glas- oder Kunststoffplatten oder Kunststoffolien, von denen mindestens eine Platte oder Folie, vorzugsweise beide Platten oder Folien auf jeweils einer Seite mit einer elektrisch leitfähigen Beschichtung versehen sind, von denen wenigstens eine Plane oder Folie und ihre leitfähige Beschichtung transparent sind, von denen die andere verspiegelt sein kann und von denen wenigstens bei einer der beiden Planen oder Folien die elektrisch leitfähige Schicht in getrennte, einzeln kontaktierte Flächensegmente aufgeteilt sein kann, wobei die Platten oder Folien über einen Dichtungsring auf den Seiten ihrer leitfähigen Beschichtung zusammengefügt sind, und das Volumen, gebildet aus den beiden Planen oder Folien und dem Dichtungsring, mit einem elektrochromen Medium gefüllt ist, das ein Paar elektrochromer Substanzen $OX_2$ und $RED_1$ enthält, dadurch gekennzeichnet, dass $RED_1$ einer der Formeln

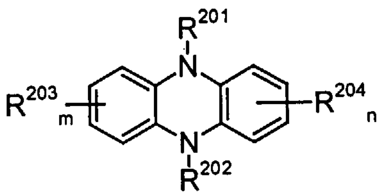

(CC),

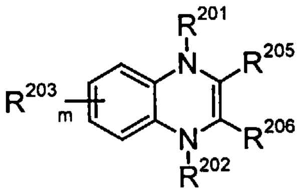

(CCI),

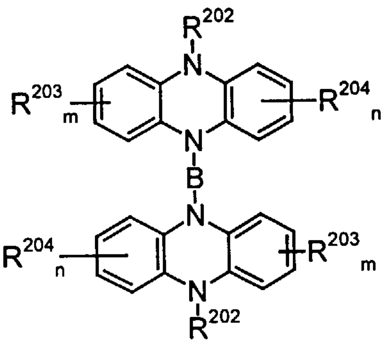

(CCII) oder

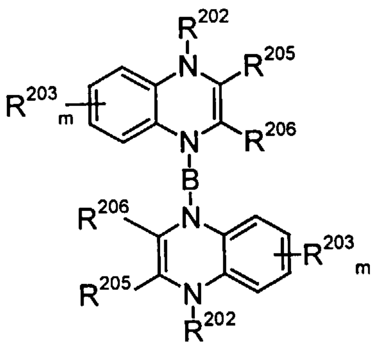

(CCIII)

entspricht, worin

$R^{201}$ für Aryl steht,

$R^{202}$ für Alkyl, Cycloalkyl, Alkenyl, Aralkyl oder Aryl steht,

B für eine bivalente Brücke steht,

$R^{203}$ bis $R^{206}$ unabhängig voneinander für Wasserstoff, Halogen, Alkyl, Alkoxy, Cyano oder Aryl stehen,

m und n unabhängig voneinander für eine ganze Zahl von 1 bis 4 stehen oder

zwei benachbarte $R^{203}$ und $R^{204}$ unabhängig voneinander für einen bivalenten Rest -CH=CH=CH=CH- stehen, wenn m bzw. n für ≥ steht.

Solche Dihydrophenazine sind beispielsweise aus H. Gilman und J. J. Dietrich, J. Amer. Chem. Soc. **79** (1957) 6178 bekannt oder lassen sich in analoger Weise herstellen.

Bevorzugte Dihydronaphthazine und Dihydrophenazine der Formeln (CC) bis (CCIII) sind solche, worin

$R^{201}$ für $C_6$- bis $C_{10}$-Aryl steht,

$R^{202}$ für $C_1$- bis $C_{12}$-Alkyl, $C_3$- bis $C_7$-Cycloalkyl, $C_2$- bis $C_{12}$-Alkenyl, $C_7$- bis $C_{16}$-Aralkyl oder $C_6$- bis $C_{10}$-Aryl steht,

B für $-(CH_2)_p-$, $-(CH_2)-(O-CH_2)_q-O-CH_2-$ oder $-(CH_2)_r-C_6H_4-(CH_2)_s-$ steht, wobei die $CH_2$-Gruppen durch Methyl substituiert sein können, $R^{203}$ bis $R^{206}$ unabhängig voneinander für Wasserstoff, Halogen, $C_1$- bis $C_4$-Alkyl, $C_1$-bis $C_4$-Alkoxy, Cyano oder $C_6$- bis $C_{10}$-Aryl stehen,

m und n unabhängig voneinander für eine ganze Zahl von 1 bis 4 stehen oder

zwei benachbarte $R^{203}$ und $R^{204}$ unabhängig voneinander für einen bivalenten Rest -CH=CH-CH=CH- stehen, wenn m bzw. n für ≥ steht,

p für eine ganze Zahl von 2 bis 20 steht und

q, r und s unabhängig voneinander für eine ganze Zahl von 0 bis 10 stehen.

Besonders bevorzugte Dihydronaphthazine und Dihydrophenazine der Formeln (CC) bis (CCIII) sind solche, worin

$R^{201}$ für Phenyl steht, das gegebenenfalls bis zu drei Methyl-, Methoxy-, Chlor-, Brom- oder Cyano-Reste tragen kann,

$R^{202}$ für gegebenenfalls verzweigtes $C_1$- bis $C_8$-Alkyl, Cyclopentyl, Cyclohexyl, Benzyl, Phenethyl, Phenylpropyl oder Phenyl steht, wobei diese Reste gegebenenfalls bis zu drei Methyl-, Methoxy-, Chlor-, Brom- oder Cyano-Reste tragen können,

B für $-(CH_2)_p-$ steht,

$R^{203}$ bis $R^{206}$ unabhängig voneinander für Wasserstoff, Chlor, Brom, Methyl, Ethyl, Propyl, Butyl, Methoxy, Ethoxy, Cyano oder Phenyl stehen,

m und n unabhängig voneinander für eine ganze Zahl von 1 bis 2 stehen oder

zwei benachbarte $R^{203}$ und $R^{204}$ unabhängig voneinander für einen bivalenten Rest -CH=CH-CH=CH- stehen, wenn m bzw. n für 2 steht und

p für eine ganze Zahl von 2 bis 10 steht.

In ganz besonders bevorzugter Form enthält die erfindungsgemäße elektrochrome Vorrichtung ein $RED_1$ der Formel (CC).

Bevorzugt sind solche Dihydrophenazine der Formel (CC), worin

$R^{201}$ für Phenyl steht,

$R^{202}$ für Methyl, Ethyl, Propyl, Butyl, Phenylpropyl oder Phenyl, besonders bevorzugt Phenyl steht,

$R^{203}$ und $R^{204}$ für Wasserstoff stehen und

m und n 1 bedeuten.

In ebenfalls ganz bevorzugter Weise enthält die erfindungsgemäße elektrochrome Vorrichtung ein $RED_1$ der Formel (CCII).

Bevorzugt sind solche Dihydrophenazine der Formel (CCII), worin

$R^{202}$ für Phenyl steht,

B für $-(CH_2)_p-$ steht,

$R^{203}$ und $R^{204}$ für Wasserstoff stehen,

m und n 1 bedeuten und

p für eine ganze Zahl von 2 bis 6 steht.

Die erfindungsgemäße elektrochrome Vorrichtung enthält neben elektrochromen Substanzen $RED_1$ der Formeln (CC) bis (CCIII) mindestens eine elektrochrome Substanz $OX_2$. Sie kann aber auch weitere $RED_1$ und/oder $OX_2$ enthalten.

Durch Auswahl der elektrochromen Verbindungen $RED_1$ und $OX_2$ und/oder Mischungen davon lassen sich beliebige monochrome Farbtöne einstellen. Für eine polychrome Farbdarstellung können zwei oder mehrere solcher elektrochromer Vorrichtungen flächig aufeinander gelegt werden, wobei jede dieser Vorrichtungen einen anderen Farbton erzeugen kann. Vorzugsweise wird ein solcher Stapel so aufgebaut, dass die sich berührenden Vorrichtungen eine lichtdurchlässige Platte gemeinsam haben, die dann auch auf beiden Seiten leitfähig beschichtet ist und je nach Ausführung in Segmente unterteilt ist. Beispielsweise besteht dann ein Stapel aus drei elektrochromen Vorrichtungen aus mindestens vier Platten. Durch Einschalten von Segmenten in verschiedenen dieser gestapelten Vorrichtungen lassen sich mehrfarbige Anzeigen realisieren. Werden hintereinander liegende Segmente verschiedener solcher Vorrichtungen eingeschaltet, erhält man Mischfarben. So lassen sich im Rahmen einer Trichromie beliebige Farben darstellen, also beispielsweise bunte Bilder.

Bevorzugt sind solche erfindungsgemäßen elektrochromen Vorrichtungen, die eine oxidierbare Substanz $RED_1$ der Formeln (CC) bis (CCIII) sowie eine reduzierbare Substanz $OX_2$ sowie gegebenenfalls weitere oxidierbare und/oder reduzierbare Substanzen enthält, wobei

a) die reduzierbare Substanz mindestens eine, vorzugsweise wenigstens zwei chemisch reversible Reduktionswellen im cyclischen Voltammogramm und die oxidierbare Substanz entsprechend mindestens eine, vorzugsweise wenigstens zwei chemisch reversible Oxidationswellen besitzen, oder

b) die reduzierbare Substanz und die oxidierbare Substanz über eine Brücke B kovalent aneinander gebunden sind, oder

c) als reduzierbare und/oder oxidierbare Substanz solche ausgewählt sind, bei denen der reversible Übergang zwischen der oxidierbaren Form und der reduzierbaren Form oder umgekehrt mit dem Bruch bzw. dem Aufbau einer σ-Bindung verbunden ist, oder

d) die reduzierbare Substanz und/oder die oxidierbare Substanz Metallsalze oder Metallkomplexe sind von solchen Metallen, die in mindestens zwei Oxidationsstufen existieren, oder

e) die reduzierbare und/oder oxidierbare Substanz Oligo- und Polymere sind, die mindestens eines der genannten Redoxsysteme, aber auch Paare solcher Redoxsysteme, wie sie unter a) bis d) definiert sind, enthalten, oder

als reduzierbare und/oder oxidierbare Substanz Mischungen der in a) bis e) beschriebenen Substanzen eingesetzt werden.

Im Sinne der Erfindung geeignete $OX_2$ und weitere $RED_1$ sind solche Substanzen, die bei ihrer Reduktion bzw. Oxidation an der Kathode bzw. Anode in dem genannten Lösungsmittel Produkte $RED_2$ und $OX_1$ liefern, die keine chemische Folgereaktion eingehen, sondern komplett wieder zu $OX_2$ und $RED_1$ oxidiert bzw. reduziert werden können.

Geeignete reduzierbare Substanzen $OX_2$ sind beispielsweise

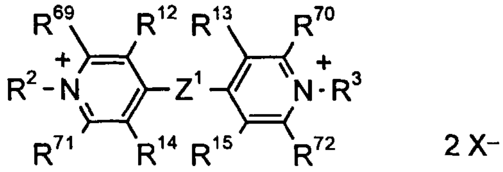

(I),

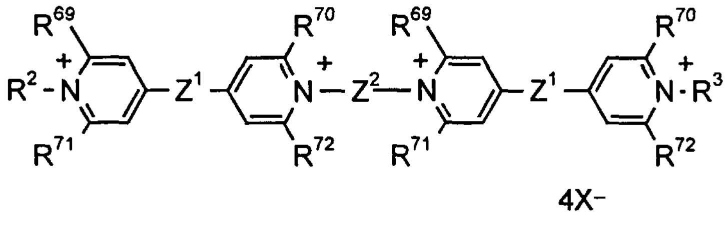

(II),

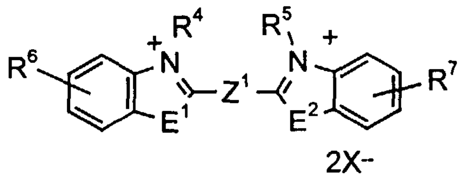

(III),

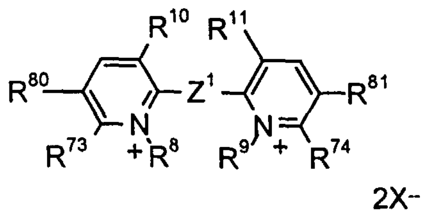

(IV),

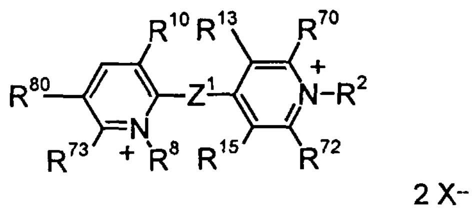

(V),

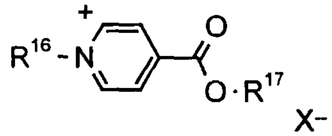

(VI),

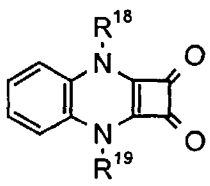

(VII),

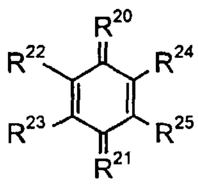

(VIII),

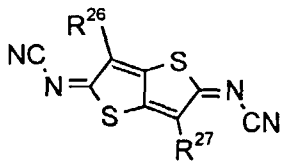

(IX),

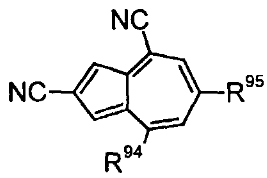

(X),

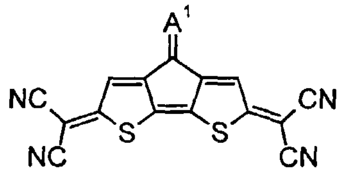

(XI),

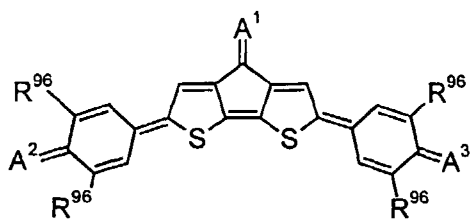

(XII),

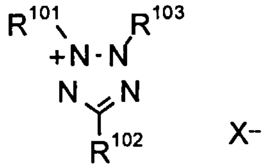

(CI),

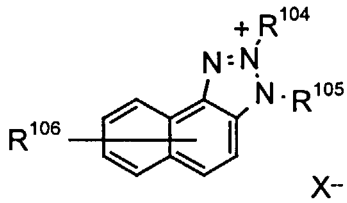

(CII),

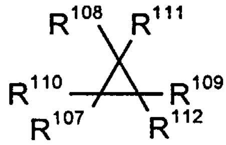

(CIII),

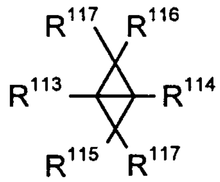

(CIV),

worin

$R^2$ bis $R^5$, $R^8$, $R^9$, $R^{16}$ bis $R^{19}$ unabhängig voneinander $C_1$- bis $C_{18}$-Alkyl, $C_2$- bis $C_{12}$-Alkenyl, $C_4$- bis $C_7$-Cycloalkyl, $C_7$- bis $C_{15}$-Aralkyl oder $C_6$- bis $C_{10}$-Aryl bedeuten oder

$R^4$; $R^5$ bzw. $R^8$; $R^9$ gemeinsam eine $-(CH_2)_2$- oder $-(CH_2)_3$-Brücke bilden können,

$R^6$, $R^7$ und $R^{22}$ bis $R^{25}$ unabhängig voneinander Wasserstoff, $C_1$- bis $C_4$-Alkyl, $C_1$- bis $C_4$-Alkoxy, Halogen, Cyan, Nitro oder $C_1$- bis $C_4$-Alkoxycarbonyl bedeuten oder

$R^{22}$; $R^{23}$ und/oder $R^{24}$; $R^{25}$ eine -CH=CH-CH=CH-Brücke bilden können,

$R^{10}$; $R^{11}$, $R^{10}$; $R^{13}$, $R^{12}$; $R^{13}$ und $R^{14}$; $R^{15}$ unabhängig voneinander Wasserstoff oder paarweise eine $-(CH_2)_2$-, $-(CH_2)_3$- oder -CH=CH-Brücke bedeuten,

$R^{20}$ und $R^{21}$ unabhängig voneinander O, N-CN, $C(CN)_2$ oder N-$C_6$- bis $C_{10}$-Aryl bedeuten,

$R^{26}$ und $R^{27}$ Wasserstoff $C_1$ - bis $C_4$-Alkyl, $C_1$- bis $C_4$-Alkoxy, Halogen, Cyan, Nitro, $C_1$- bis $C_4$-Alkoxycarbonyl oder $C_6$- bis $C_{10}$-Aryl bedeuten,

$R^{69}$ bis $R^{74}$, $R^{80}$ und $R^{81}$ unabhängig voneinander Wasserstoff oder $C_1$- bis $C_6$-Alkyl bedeuten oder

$R^{69}$; $R^{12}$, $R^{70}$; $R^{13}$, $R^{73}$; $R^{80}$ und/oder $R^{74}$; $R^{81}$ gemeinsam eine -CH=CH-CH=CH-Brücke bilden,

$E^1$ und $E^2$ unabhängig voneinander O, S, $NR^1$ oder $C(CH_3)_2$ bedeuten oder

$E^1$ und $E^2$ gemeinsam eine $-N-(CH_2)_2-N-$Brücke bilden,

$R^1$ $C_1$- bis $C_{18}$-Alkyl, $C_2$- bis $C_{12}$-Alkenyl, $C_4$- bis $C_7$-Cycloalkyl, $C_7$- bis $C_{15}$-Aralkyl, $C_6$- bis $C_{10}$-Aryl bedeutet,

$Z^1$ eine direkte Bindung, -CH=CH-, $-C(CH_3)=CH-$, -C(CN)=CH-, -CCl=CCl-, -C(OH)=CH-, -CCl=CH-, -C≡C-,-CH=N-N=CH-, $-C(CH_3)=N-N=C(CH_3)$-oder -CCl=N-N=CCl- bedeutet,

$Z^2$ $-(CH_2)$-oder $-CH_2-C_6H_4-CH_2-$ bedeutet,

r eine ganze Zahl von 1 bis 10 bedeutet,

$R^{94}$ und $R^{95}$ unabhängig voneinander Wasserstoff oder Cyano bedeuten,

$R^{101}$ bis $R^{105}$ unabhängig voneinander $C_6$- bis $C_{10}$-Aryl oder einen ggf. benzanellierten aromatischen oder quasiaromatischen fünf- oder sechsgliedrigen heterocyclischen Ring bedeuten,

$R^{107}$, $R^{109}$, $R^{113}$ und $R^{114}$ unabhängig voneinander einen Rest der Formeln (CV) bis (CVII)

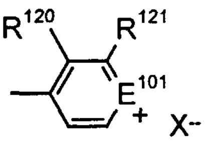

(CV),

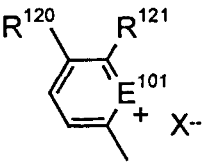

(CVI),

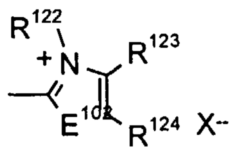

(CVII)

bedeuten,

$R^{108}$, $R^{115}$ und $R^{116}$ unabhängig voneinander $C_6$- bis $C_{10}$-Aryl oder einen Rest der Formel (CV) bedeuten,

$R^{110}$ bis $R^{112}$, $R^{117}$ und $R^{118}$ unabhängig voneinander Wasserstoff, $C_1$- bis $C_4$-Alkyl, Halogen oder Cyano bedeuten,

$E^{101}$ und $E^{102}$ unabhängig voneinander O, S oder $N-R^{119}$ bedeuten,

$R^{119}$ und $R^{122}$ unabhängig voneinander $C_1$- bis $C_{18}$-Alkyl, $C_2$- bis $C_8$-Alkenyl, $C_4$- bis $C_7$-Cycloalkyl, $C_7$- bis $C_{15}$-Aralkyl oder $C_6$- bis $C_{10}$-Aryl bedeuten,

$R^{106}$, $R^{120}$, $R^{121}$, $R^{123}$ und $R^{124}$ unabhängig voneinander Wasserstoff, $C_1$- bis $C_4$-Alkyl, $C_1$- bis $C_4$-Alkoxy, Halogen, Cyano, Nitro oder $C_1$- bis $C_4$-Alkoxycarbonyl bedeuten oder

$R^{120}$, $R^{121}$ bzw. $R^{123}$, $R^{124}$ gemeinsam eine -CH=CH-CH=CH-Brücke bilden,

$A^1$, $A^2$ und $A^3$ unabhängig voneinander O oder $C(CN)_2$ bedeuten,

$R^{96}$ Wasserstoff, Phenyl oder tert.-Butyl bedeutet und

$X^-$ ein unter den Bedingungen redox-inertes Anion bedeutet.

Geeignete oxidierbare Substanzen $RED_1$ sind beispielsweise

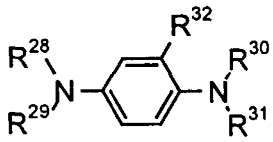

(XX),

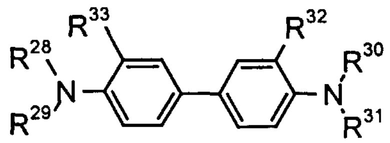

(XXI),

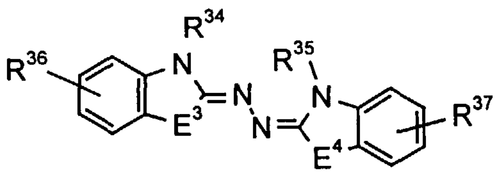

(XXII),

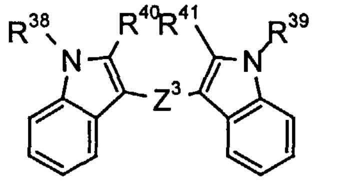

(XXIII),

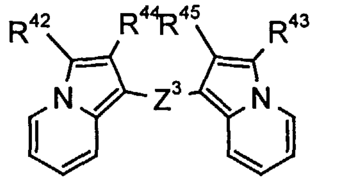

(XXIV),

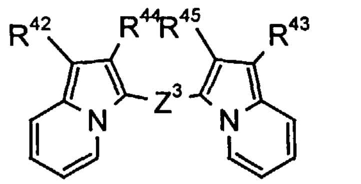

(XXV),

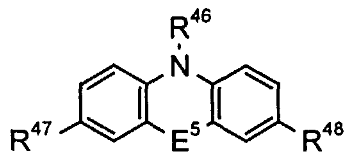

(XXVI),

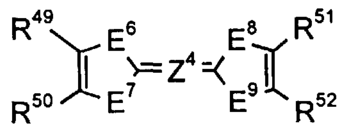

(XXVII),

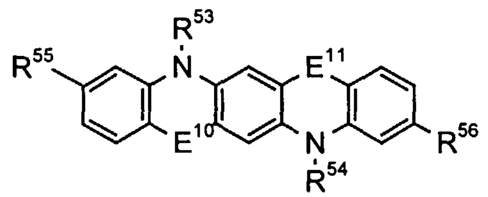

(XXVIII),

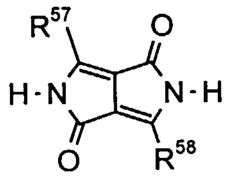

(XXIX),

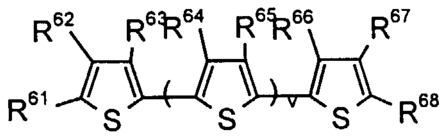

(XXX),

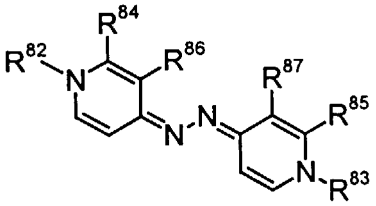

(XXXI),

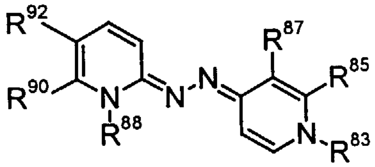

(XXXII),

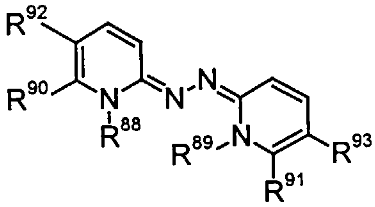

(XXXIII),

worin

$R^{28}$ bis $R^{31}$, $R^{34}$, $R^{35}$, $R^{38}$, $R^{39}$, $R^{46}$, $R^{53}$ und $R^{54}$ unabhängig voneinander $C_1$- bis $C_{18}$-Alkyl, $C_2$- bis $C_{12}$-Alkenyl, $C_4$- bis $C_7$-Cycloalkyl, $C_7$- bis $C_{15}$-Aralkyl oder $C_6$- bis $C_{10}$-Aryl bedeuten,

$R^{32}$, $R^{33}$, $R^{36}$, $R^{37}$, $R^{40}$, $R^{41}$, $R^{42}$ bis $R^{45}$, $R^{47}$, $R^{48}$, $R^{49}$ bis $R^{52}$ und $R^{55}$ bis $R^{58}$ unabhängig voneinander Wasserstoff, $C_1$- bis $C_4$-Alkyl, $C_1$- bis $C_4$-Alkoxy, Halogen, Cyan, Nitro, $C_1$- bis $C_4$-Alkoxycarbonyl, $C_6$- bis $C_{10}$-Aryl bedeuten
und

$R^{57}$ und $R^{58}$ zusätzlich einen aromatischen oder quasiaromatischen fünf- oder sechsgliedrigen heterocyclischen Ring, der gegebenenfalls benzanneliert ist, bedeuten und $R^{48}$ zusätzlich $NR^{75}R^{76}$ bedeutet oder

$R^{49}$; $R^{50}$ und/oder $R^{51}$; $R^{52}$ eine $-(CH_2)_3-$, $-(CH_2)_4-$, $-(CH_2)_5-$ oder -CH=CH-CH=CH-Brücke bilden,

$Z^3$ eine direkte Bindung, eine -CH=CH- oder -N=N-Brücke bedeutet,

$=Z^4=$ eine direkte Doppelbindung, eine =CH-CH= oder =N-N=-Brücke bedeutet,

$E^3$ bis $E^5$, $E^{10}$ und $E^{11}$ unabhängig voneinander O, S, $NR^{59}$ oder $C(CH_3)_2$ bedeuten und

$E^5$ zusätzlich C=O oder $SO_2$ bedeutet,

$E^3$ und $E^4$ unabhängig voneinander zusätzlich -CH=CH- bedeuten können,

$E^6$ bis $E^9$ unabhängig voneinander S, Se oder $NR^{59}$ bedeuten,

$R^{59}$, $R^{75}$ und $R^{76}$ unabhängig voneinander $C_1$- bis $C_{12}$-Alkyl, $C_2$- bis $C_8$-Alkenyl, $C_4$-bis $C_7$-Cycloalkyl, $C_7$- bis $C_{15}$-Aralkyl, $C_6$- bis $C_{10}$-Aryl bedeuten,
und

$R^{75}$ zusätzlich Wasserstoff bedeutet oder $R^{75}$ und $R^{76}$ in der Bedeutung von $NR^{75}R^{76}$ gemeinsam mit dem N-Atom, an das sie gebunden sind, einen fünf- oder sechsgliedrigen Ring bedeuten, der gegebenenfalls weitere Heteroatome enthält,

$R^{61}$ bis $R^{68}$ unabhängig voneinander Wasserstoff, $C_1$- bis $C_6$-Alkyl, $C_1$- bis $C_4$-Alkoxy, Cyan, $C_1$- bis $C_4$-Alkoxycarbonyl oder $C_6$- bis $C_{10}$-Aryl bedeuten und

$R^{61}$; $R^{62}$ und $P^{67}$; $R^{68}$ unabhängig voneinander zusätzlich eine $-(CH_2)_3-$, $-(CH_2)_4-$ oder -CH=CH-CH=CH-Brücke bilden oder

$R^{62}$; $R^{63}$, $R^{64}$; $R^{65}$ und $R^{66}$; $R^{67}$ eine $-O-CH_2CH_2-O-$ oder $-O-CH_2CH_2CH_2-O-$Brücke bilden,

v eine ganze Zahl zwischen 0 und 100 bedeutet,

$R^{82}$, $R^{83}$, $R^{88}$ und $R^{89}$ unabhängig voneinander $C_1$- bis $C_{18}$-Alkyl, $C_2$- bis $C_{12}$-Alkenyl, $C_4$- bis $C_7$-Cycloalkyl, $C_7$- bis $C_{15}$-Aralkyl oder $C_6$- bis $C_{10}$-Aryl bedeuten,

$R^{84}$ bis $R^{87}$ und $R^{90}$ bis $R^{93}$ unabhängig voneinander Wasserstoff oder $C_1$- bis $C_6$-Alkyl bedeuten oder

$R^{84}$; $R^{86}$, $R^{85}$; $R^{87}$, $R^{90}$; $R^{92}$ und/oder $R^{91}$; $R^{93}$ gemeinsam eine -CH=CH-CH=CH-Brücke bilden.

Ebenfalls geeignet als $RED_1$ sind Anionen wie z.B. $I^-$, $I_3^-$, $Br^-$, $SCN^-$.

Über eine Brücke B verknüpfte, gegebenenfalls oligo- oder polymere Redoxsysteme sind beispielsweise solche der Formel

$$Y-[-(-B-Z-)_a-(-B-Y-)_b-]_c-B-Z \qquad (L),$$

worin

Y und Z unabhängig voneinander für einen Rest $OX_2$ oder $RED_1$ stehen, wobei aber entweder mindestens ein Y für $OX_2$ und mindestens ein Z für $RED_1$ steht oder Y und Z für $OX_2$ stehen,
wobei

$OX_2$ für den Rest eines reversibel elektrochemisch reduzierbaren Redoxsystems steht, und

$RED_1$ für den Rest eines reversibel elektrochemisch oxidierbaren Redoxsystems steht,

B für ein Brückenglied steht,

c für eine ganze Zahl von 0 bis 1000 steht, und

a und b unabhängig voneinander für eine ganze Zahl von 0 bis 100 stehen.

Vorzugsweise ist $(a+b) \cdot c \leq 10.000$ .

Hierbei ist unter reversibel elektrochemisch reduzierbar oder oxidierbar gemeint, dass die Elektronenübertragung ohne oder auch mit Änderung des σ-Gerüsts erfolgen kann ganz im Sinne der oben genannten Definition der erfindungsgemäßen $OX_2$ und $RED_1$.

Insbesondere sind mit den elektrochromen Verbindungen der Formel (L) solche der Formeln

$$OX_2\text{-B-}RED_1 \qquad (La),$$

$$OX_2\text{-B-}RED_1\text{-B-}OX_2 \qquad (Lb),$$

$$RED_1\text{-B-}OX_2\text{-B-}RED_1 \qquad (Lc),$$

$$OX_2\text{-(B-}RED_1\text{-B-}OX_2)_d\text{-B-}RED_1 \qquad (Ld)$$

oder

$$OX_2\text{-(B-}OX_2)_e\text{-B-}OX_2 \qquad (Le)$$

gemeint,
worin

$OX_2$, $RED_1$ und B die oben angegebene Bedeutung haben,

d für eine ganze Zahl von 1 bis 5 steht und

e für eine ganze Zahl von 0 bis 5 steht.

Mit $OX_2$ und $RED_1$ in den Formeln (L) und (La) bis (Le) sind insbesondere Reste der oben beschriebenen Redoxsysteme der Formeln (I) bis (X), (CI) bis CIV) und (XX) bis (XXXIII) gemeint, wobei die Bindung zum Brückenglied B über einen der Reste $R^2$ bis $R^{19}$, $R^{22}$ bis $R^{27}$, $R^{28}$ bis $R^{58}$, $R^{61}$, $R^{62}$, $R^{67}$, $R^{68}$, $R^{83}$, $R^{88}$, $R^{122}$ oder im Falle, dass einer der Reste $E^1$ oder $E^2$ für $NR^1$ oder einer der Reste $E^3$ bis $E^{11}$ für $NR^{59}$ oder einer der Reste $E^{101}$ bis $E^{102}$ für $NR^{119}$ steht, über $R^1$, $R^{59}$ bzw. $R^{119}$ erfolgt und die genannten Reste dann für eine direkte Bindung stehen, und

B für eine Brücke der Formeln $-(CH_2)_n-$ oder $-[Y^1{}_s(CH_2)_m-Y^2]_o-(CH_2)_p-Y^3{}_q-$ steht, die durch $C_1$- bis $C_4$-Alkyl, $C_1$- bis $C_4$-Alkoxy, Halogen oder Phenyl substituiert sein kann,

$Y^1$ bis $Y^3$ unabhängig voneinander für O, 5, $NR^{60}$, COO, CONH, NHCONH, Cyclopentandiyl, Cyclohexandiyl, Phenylen oder Naphthylen stehen,

$R^{60}$ $C_1$- bis $C_6$-Alkyl, $C_2$- bis $C_6$-Alkenyl, $C_4$- bis $C_7$-Cycloalkyl, $C_7$- bis $C_{15}$-Ar-alkyl oder $C_6$- bis $C_{10}$-Aryl bedeutet,

n eine ganze Zahl von 1 bis 12 bedeutet,

m und p unabhängig voneinander eine ganze Zahl von 0 bis 8 bedeuten,

o eine ganze Zahl von 0 bis 6 bedeutet und

q und s unabhängig voneinander 0 oder 1 bedeuten.

In ganz besonderem Maße sind mit $OX_2$ und $RED_1$ in den Formeln (L) und (La) bis (Le) Reste der oben beschriebenen Redoxsysteme der Formeln (I), (V), (XX), (XXII), (XXIII), (XXV), (XXVI) und (XXXIII) gemeint.

In einem anderen Typ oligo- oder polymerer Systeme können die Gruppierungen $OX_2$ und/oder $RED_1$ auch beispielsweise als Seitenketten an einer Hauptgruppe, beispielsweise einem Poly(meth)acrylat, Silikon, Polycarbonat, Polyurethan, Polyharnstoff, Polyester, Polyamid, Cellulose oder anderen oligo- oder polymeren Systemen angebunden sein.

Beispiele für Metallsalze oder Metallkomplexe, die als $OX_2$ oder $RED_1$ eingesetzt werden können, sind

$Fe^{3+/2+}$, $Ni^{3+/2+}$, $Co^{3+/2+}$, $Cu^{2+/+}$, $[Fe(CN)_6]^{3-/4-}$, $Fe_4[Fe(CN)_6]_3^{0/4-}$, $[Co(CN)_6]^{3-/4-}$, $[Fe(Cyclopentadienyl)_2]^{0/+}$, $Lu(Pc)^{2+\text{ bis }2-}$ (Pc = Phthalocyanin), $Fe[Fe(CN)_6]^{0/1-}$.

Als Gegenionen für Metallionen und kationische Komplexe kommen alle redoxinerten Anionen $X^-$, wie sie später noch genauer beschrieben werden, in Frage, als Gegenionen der anionischen Komplexe alle redox-inerten Kationen $M'^+$ in Frage, beispielsweise Alkalimetalle oder quaternierte Ammoniumsalze wie $Na^+$, $K^+$, $N(CH_3)_4^+$, $N(C_4H_9)_4^+$, $C_6H_5CH_2N(CH_3)_3^+$ und andere.

Ebenfalls bevorzugt ist eine elektrochrome Vorrichtung, die Mischungen der oben allgemein und bevorzugt genannten elektrochromen Substanzen enthält. Beispiele für solche Mischungen sind (I) + (CI) + (CC), (I) + (IV) + (CC) + (XXII), (La) + (I) + (CC) + (XXVI), ohne dass dadurch irgendeine Einschränkung ausgedrückt werden soll.

Die Mischungsverhältnisse sind in weiten Grenzen variabel. Sie erlauben die Optimierung eines gewünschten Farbtons oder Schwärzegrades und/oder die Optimierung der gewünschten Dynamik der Vorrichtung.

In den oben genannten Substituentenbedeutungen sind Alkylreste, auch abgewandelte, beispielsweise Alkoxy- oder Aralkylreste, vorzugsweise solche mit 1 bis 12 C-Atomen, insbesondere mit 1 bis 8 C-Atomen, sofern nichts anderes angegeben ist. Sie können geradkettig oder verzweigt sein und gegebenenfalls weitere Substituenten tragen wie $C_1$- bis $C_4$-Alkoxy, Fluor, Chlor, Hydroxy, Cyano, $C_1$- bis $C_4$-Alkoxy-carbonyl oder COOH.

Unter Cycloalkylresten werden vorzugsweise solche mit 3 bis 7 C-Atomen, insbesondere mit 5 oder 6 C-Atomen verstanden.

Alkenylreste sind vorzugsweise solche mit 2 bis 8 C-Atomen, insbesondere 2 bis 4 C-Atomen.

Arylreste, auch solche in Aralkylresten, sind Phenyl oder Naphthylreste, insbesondere Phenylreste. Sie können durch 1 bis 3 der folgenden Reste substituiert sein: $C_1$- bis $C_6$-Alkyl, $C_1$- bis $C_6$-Alkoxy, Fluor, Chlor, Brom, Cyano, Hydroxy, $C_1$- bis $C_6$-Alkoxycarbonyl oder Nitro. Zwei benachbarte Reste können auch einen Ring bilden.

Unter gegebenenfalls benzanellierten aromatischen oder quasiaromatischen fünf- oder sechsgliedrigen heterocyclischen Ringen werden insbesondere Imidazol, Benzimidazol, Oxazol, Benzoxazol, Thiazol, Benzthiazol, Indol, Pyrazol, Triazol, Thiophen, Isothiazol, Benzisothiazol, 1,3,4- oder 1,2,4-Thiadiazol, Pyridin, Chinolin, Pyrimidin und Pyrazin verstanden. Sie können durch 1 bis 3 der folgenden Reste substituiert sein: $C_1$- bis $C_6$-Alkyl, $C_1$- bis $C_6$-Alkoxy, Fluor, Chlor, Brom, Cyano, Nitro, Hydroxy, Mono- oder Di-$C_1$- bis $C_6$-alkylamino, $C_1$- bis $C_6$-Alkoxycarbonyl, $C_1$- bis $C_6$-Alkylsulfonyl, $C_1$- bis $C_6$- Alkanoylamino, Phenyl oder Naphthyl. Zwei benachbarte Reste können auch einen Ring bilden.

Die elektrochromen Substanzen sind entweder bekannt (Topics in Current Chemistry, Vol. 92, S. 1-44, (1980), Angew. Chem. 90, 927 (1978), Adv. Mater. 3, 225, (1991), DE-OS 3.917.323, J. Am. Chem. Soc. 117, 8528 (1995), J. C. S. Perkin II 1990, 1777, DE-OS 4.435.211, EP-A 476.456, EP-A 476.457, DE-OS 4.007.058, J. Org. Chem. 57, 1849 (1992) und J. Am. Chem. Soc. 99, 6120, 6122 (1977) oder lassen sich analog herstellen. Die Verbindungen der Formel (L) sind ebenfalls bekannt (WO 97/30134).

Synthetisch bedingte Ionen wie Bromid werden im Anschluss gegen redox-inerte Ionen ausgetauscht.

Besonders bevorzugt sind neben den erfindungsgemäßen oxidierbaren Verbindungen der Formel (CC) oder (CCII) die reduzierbaren elektrochromen Verbindungen der Formeln (I), (II), (III), (IV), (V).

Ganz besonders bevorzugt sind neben den erfindungsgemäßen oxidierbaren Verbindungen der Formel (CC) oder (CCII) die reduzierbaren elektrochromen Verbindungen der Formeln (I), (IV), (V), worin

$R^2$, $R^3$, $R^8$ und $R^9$ unabhängig voneinander Methyl, Ethyl, Propyl, Butyl, Pentyl, Hexyl, Heptyl, Benzyl, Phenethyl, Phenylpropyl, Phenyl, 2-Methylphenyl oder 2,6-Dimethylphenyl bedeuten oder

$R^8$ und $R^9$ gemeinsam eine $-(CH_2)_2-$ oder $-(CH_2)_3-$Brücke bilden,

$R^{10}$ bis $R^{15}$ Wasserstoff bedeuten,

$R^{69}$ bis $R^{73}$, $R^{80}$ und $R^{81}$ unabhängig voneinander Wasserstoff oder Methyl bedeuten oder

$R^{12}$; $R^{69}$, $R^{13}$; $R^{70}$, $R^{73}$; $R^{80}$ und/oder $R^{74}$; $R^{81}$ eine -CH=CH-CH=CH-Brücke bilden,

$Z^1$ eine direkte Bindung oder -CH=CH- bedeutet und

$X^-$ ein unter den Bedingungen redox-inertes Anion bedeutet,

wobei die Alkylreste verzweigt sein können, beispielsweise 2-Butyl, 1-Phenyl-2-propyl.

Im Sinne der Erfindung ganz herausragend geeignet sind die elektrochromen Verbindungen der Formel (I), worin

$R^2$ und $R^3$ gleich sind und Methyl, Ethyl, Butyl, Heptyl oder Phenylpropyl bedeuten,

$R^{12}$ bis $R^{15}$ und $R^{69}$ bis $R^{72}$ Wasserstoff bedeuten,

$Z^1$ eine direkte Bindung bedeutet und

$X^-$ ein redoxinertes Anion oder $I^-$ bedeutet.

Die erfindungsgemäße lichtgeschützte elektrochrome Vorrichtung enthält in ihrem elektrochromen Medium vorzugsweise mindestens ein Lösungsmittel, in dem die elektrochromen Substanzen, gegebenenfalls ein Leitsalz und gegebenenfalls weitere Zusätze gelöst sind. Das Lösungsmittel kann auch gelförmig verdickt sein, beispielsweise durch Polyelektrolyte, poröse Feststoffe oder Nanopartikel mit großer aktiver Oberfläche.

Geeignete Lösungsmittel sind alle unter den gewählten Spannungen redox-inerten Lösungsmittel, die keine Elektrophile oder Nukleophile abspalten können oder selber als ausreichend starke Elektrophile oder Nukleophile reagieren und so mit den farbigen Radikalionen reagieren könnten. Beispiele sind Propylencarbonat, $\gamma$-Butyrolacton, Acetonitril, Propionitril, Benzonitril, Glutaronitril, Methylglutarnitril, 3,3'-Oxydipropionitril, Hydroxypropionitril, Dimethylformamid, N-Methylpyrrolidon, Sulfolan, 3-Methylsulfolan oder Mischungen davon. Bevorzugt sind Propylencarbonat, Benzonitril und Mischungen untereinander oder mit Glutaronitril oder 3-Methylsulfolan. Insbesondere bevorzugt ist Propylencarbonat. Ebenfalls insbesondere bevorzugt ist Benzonitril.

Die elektrochrome Lösung kann mindestens ein inertes Leitsalz enthalten. Insbesondere wenn wenigstens eine der Substanzen des Redoxpaares $RED_1/OX_2$ ionischer Natur ist, kann auf den Zusatz eines Leitsalzes verzichtet werden.

Als inertes Leitsalz sind Lithium-, Natrium- und Tetraalkylammoniumsalze geeignet, insbesondere letztere. Die Alkylgruppen können zwischen 1 und 18 C-Atome aufweisen und gleich oder verschieden sein. Bevorzugt ist Tetrabutylammonium. Als Anionen zu diesen Salzen, aber auch als Anionen $X^-$ in den Formeln (I) bis (VI), (CI), (CII) und (CV) bis (CVII) und in den Metallsalzen kommen alle redox-inerten, farblosen Anionen in Frage.

Beispiele sind Tetrafluoroborat, Tetraphenylborat, Cyano-triphenylborat, Tetramethoxyborat, Tetrapropoxyborat, Tetraphenoxyborat, Perchlorat, Chlorid, Nitrat, Sulfat, Phosphat, Methansulfonat, Ethansulfonat, Tetradecansulfonat, Pentadecansulfonat, Trifluormethansulfonat, Perfluorbutansulfonat, Perfluoroctansulfonat, Benzolsulfonat, Chlorbenzolsulfonat, Toluolsulfonat, Butylbenzolsulfonat, tert. Butylbenzolsulfonat, Dodecylbenzolsulfonat, Trifluormethylbenzolsulfonat, Hexafluorophosphat, Hexafluoroarsenat, Hexafluorosilicat, 7,8- oder 7,9-Dicarbanido-undecaborat(-1) oder (-2), die gegebenenfalls an den B- und/oder C-Atomen durch eine oder zwei Methyl-, Ethyl-, Butyl- oder Phenyl-Gruppen substituiert sind, Dodecahydro-dicarbadodecaborat(-2) oder B-Methyl-C-phenyl-dodecahydro-dicarbadodecaborat(-1).

Ebenfalls geeignet, auch als Anionen $X^-$ in den Formeln (I) bis (VI), (CI), (CII) und (CV) bis (CVII) und in den Metallsalzen, sind die oben erwähnten Anionen, die auch die Rolle eines $RED_1$ übernehmen können, beispielsweise $I^-$, $I_3^-$.

Die Leitsalze werden vorzugsweise im Bereich 0 bis 1 mol/l eingesetzt.

Als weitere Zusätze können Verdicker eingesetzt werden, um die Viskosität der elektroaktiven Lösung zu steuern. Das kann Bedeutung haben zur Vermeidung von Segretation, d.h. der Bildung von streifiger oder fleckiger Farbbildung bei längerem Betrieb der elektrochromen Vorrichtung im eingeschalteten Zustand, und zur Steuerung der Ausbleichgeschwindigkeit nach Abschalten des Stroms.

Als Verdicker eignen sich alle für diesen Zweck üblichen Verbindungen wie z. B. Polyacrylat, Polymethacrylat (Luctite L ®), Polycarbonat oder Polyurethan.

Als weitere Zusätze für die elektrochrome Lösung kommen zum fallweise erwünschten Schutz vor UV-Licht (< 350 nm) UV-Absorber in Frage. Beispiele sind UVINUL® 3000 (2,4-Dihydroxybenzophenon, BASF), SANDUVOR® 3035 (2-Hydroxy-4-n-octyloxybenzophenon, Clariant), Tinuvin® 571 (2-(2H-Benzotriazol-2-yl)-6-dodecyl-4-methylphenol, Ciba), Cyasorb 24™ (2,2'-Dihydroxy-4-methoxy-benzophenon, American Cyanamid Company), UVINUL® 3035 (Ethyl-2-cyano-3,3-diphenylacrylat, BASF), UVINUL® 3039 (2-Ethylhexyl-2-cyano-3,3-diphenylacrylat, BASF), UVINUL® 3088 (2-Ethylhexyl-p-methoxycinnamat, BASF), CHIMASSORB® 90 (2-Hydroxy-4-methoxy-benzophenon, Ciba).

Bevorzugt sind die vier Letztgenannten. Ebenfalls bevorzugt sind Mischungen von UV-Absorbern, beispielsweise der vier Letztgenannten. Besonders bevorzugt ist die Mischung aus UVINUL® 3039 und CHIMASSORB® 90.

Die UV-Absorber werden im Bereich 0,01 bis 2 mol/l, vorzugsweise 0,04 bis 1 mol/l eingesetzt.

Die elektrochrome Lösung enthält die elektrochromen Substanzen $OX_2$ und $RED_1$, insbesondere die der Formeln (I) bis (X) und (CC) und/oder (CCII) jeweils in einer Konzentration von mindestens $10^{-4}$ mol/l, vorzugsweise 0,001 bis 0,5mol/l. Die Gesamtkonzentration aller enthaltenen elektrochromen Substanzen liegt vorzugsweise unter 1 mol/l.

Zum Betrieb der erfindungsgemäßen elektrochromen Vorrichtung wird ein konstanter, gepulster oder in seiner Amplitude sich verändernder, beispielsweise sich sinusförmig verändernder, Gleichstrom benutzt. Die Spannung hängt ab von der gewünschten Farbtiefe, insbesondere aber von den Reduktions- bzw. Oxidationspotentialen der verwendeten $OX_2$ und $RED_1$. Solche Potentiale können beispielsweise aus Topics in Current Chemistry, Volume 92, S. 1-44, (1980) oder Angew. Chem. 90, 927 (1978) oder der dort zitierten Literatur entnommen werden. Die Differenz ihrer Potentiale ist ein Richtwert für die erforderliche Spannung, jedoch kann die elektrochrome Vorrichtung bereits bei niedrigerer oder auch mit höherer Spannung betrieben werden. In vielen Fällen, z. B. bei Verwendung von $OX_2$ = Formel (I) oder (V) und $RED_1$ = Formel (CC) liegt diese zum Betrieb nötige Potentialdifferenz ≤ V. Solche elektrochromen Vorrichtungen können deshalb in einfacher Weise mit dem Strom aus photovoltaischen Siliciumzellen versorgt werden.

Wird die Spannung abgeschaltet, geht die erfindungsgemäße elektrochrome Vorrichtung wieder in ihren ursprünglichen Zustand zurück. Diese Löschung kann erheblich beschleunigt werden, wenn die kontaktierten Segmente bzw. Platten kurzgeschlossen werden. Auch durch mehrmaliges Umpolen der Spannung, gegebenenfalls auch bei gleichzeitiger Erniedrigung der Spannung, kann die Anzeige sehr rasch gelöscht werden.

Durch Variation der Schichtdicke der elektrochromen Vorrichtung, der Viskosität der elektrochromen Lösung und/oder der Diffusions- oder Driftfähigkeit der elektrochromen Substanzen lassen sich die Einschalt- und Ausschaltzeiten der Anzeigevorrichtung in weiten Grenzen beeinflussen. So zeigen beispielsweise dünne Schichten kürzere Schaltzeiten als dicke. Es lassen sich also schnell und langsam schaltbare Vorrichtungen bauen und so den jeweiligen Einsatzzwecken optimal anpassen.

Bei langsamen Vorrichtungen, insbesondere Anzeigevorrichtungen, kann zur Aufrechterhaltung der angezeigten Information im eingeschalteten Zustand ein Stromspar- oder Refresh-Mode benutzt werden. Nach Aufbau der anzuzeigenden Information beispielsweise durch konstante oder sich mit hoher Frequenz verändernder oder gepulster Gleichspannung ausreichender Höhe wird auf gepulste oder sich verändernde Gleichspannung niedriger Frequenz umgeschaltet, wobei während der Phasen, in denen die Spannung Null beträgt, die Kontaktierung der Segmente nicht kurzgeschlossen wird. Diese niedrige Frequenz kann beispielsweise im Bereich von 1 Hz oder niedriger liegen, wobei die Dauer der Einschalt- und Ausschaltphasen nicht gleichlang zu sein brauchen, sondern beispielsweise die Ausschaltphasen deutlich länger sein können. Da sich während der Strompausen im nicht kurzgeschlossenen Zustand die Farbtiefe der angezeigten Information nur langsam abbaut, genügen relativ kurz Stromimpulse, um diese Verluste in der anschließenden Refresh-Phase wieder auszugleichen. Man erhält so ein flackerfreies Bild mit nahezu konstanter Farbtiefe, für dessen Aufrechterhaltung aber nur ein Bruchteil des Stromes benötigt wird, der bei permanentem Stromfluss anfallen würde.

Spezielle Ausführungsformen der obengenannten Typen 1 und 2 können beispielsweise die folgenden sein, die ebenfalls Gegenstand der Erfindung sind, wenn sie die erfindungsgemäßen elektrochromen Substanzen enthalten.

**Typ 1:** (unverspiegelt)

aus dem Bereich Lichtschutz/Lichtfilter: Fensterscheiben für Gebäude, Straßenfahrzeuge, Flugzeuge, Eisenbahnen, Schiffe, Dachverglasungen, Autosonnendächer, Verglasung von Gewächshäusern und Wintergärten, Lichtfilter beliebiger Art;

aus dem Bereich Sicherheit/Geheimhaltung: Trennscheiben für Raumteiler in Büros, Straßenfahrzeugen, Flugzeugen, Eisenbahnen, Sichtschutzscheiben an Bankschaltern, Türverglasungen, Scheiben für Motorrad- oder Pilotenhelme;

aus dem Bereich Design: Verglasung von Backöfen, Mikrowellengeräten, anderen Haushaltsgeräten, Möbeln.

Aus dem Bereich Anzeigen: analoge Spannungsanzeigen, als Batterietester, Tankanzeigen, Temperaturanzeigen.

**Typ 1:** (verspiegelt)

Spiegel jeglicher Art für Straßenfahrzeuge, Eisenbahnen, insbesondere plane, spärische, asphärische Spiegel und Kombinationen daraus wie spärisch/asphärisch, Spiegelverglasung in Möbeln.

**Typ 2:**

Anzeigevorrichtungen jeglicher Art, Segment- oder Matrixanzeigen für Uhren, Computer, Elektrogeräte, Elektronikgeräte wie Radios, Verstärker, Fernseher, CD-Player, Zielanzeige in Bussen und Zügen, Abfahrts- oder

Abfluganzeigen in Bahnhöfen und Flughäfen, Flachbildschirme, alle Anwendungen, die unter Typ 1 und 2 genannt sind, die mindestens eine schaltbare, statische oder variable Anzeigevorrichtung enthalten wie Trennscheiben, die Anzeigen wie ,,Bitte nicht stören,,, ,,Schalter nicht besetzt,, enthalten, Auto-Spiegel, die Anzeigen beliebiger Art enthalten, wie Anzeige der Temperatur, Störungen im Fahrzeug, beispielsweise Öltemperatur, offene Türen, Zeit, Himmelsrichtung.

Ein weiterer Gegenstand der Erfindung sind Dihydrophenazine der Formel (CC),
worin

$R^{201}$ für Aryl, insbesondere $C_6$- bis $C_{10}$-Aryl steht,

$R^{202}$ für $C_2$- bis $C_{12}$-Alkyl, $C_3$- bis $C_7$-Cycloalkyl, $C_2$- bis $C_{12}$-Alkenyl oder $C_7$-bis $C_{16}$-Aralkyl steht,

$R^{203}$ und $R^{204}$ unabhängig voneinander für Wasserstoff, Halogen, $C_1$ - bis $C_4$-Alkyl, $C_1$ - bis $C_4$-Alkoxy, Cyano oder $C_6$- bis $C_{10}$-Aryl stehen,

m und n unabhängig voneinander für eine ganze Zahl von 1 bis 4 stehen oder

zwei benachbarte $R^{203}$ und $R^{204}$ unabhängig voneinander für einen bivalenten Rest -CH=CH-CH=CH- stehen, wenn m bzw. n für ≥ 2 steht.

Insbesondere stehen in Dihydrophenazinen der Formel (CC)

$R^{201}$ für Phenyl, das gegebenenfalls bis zu drei Methyl-, Methoxy-, Chlor-, Brom- oder Cyano-Reste tragen kann,

$R^{202}$ für gegebenenfalls verzweigtes $C_2$- bis $C_8$-Alkyl, Cyclopentyl, Cyclohexyl, Benzyl, Phenethyl oder Phenylpropyl,

$R^{203}$ und $R^{204}$ unabhängig voneinander für Wasserstoff, Chlor, Brom, Methyl, Ethyl, Propyl, Butyl, Methoxy, Ethoxy, Cyano oder Phenyl,

m und n unabhängig voneinander für eine ganze Zahl von 1 bis 2 oder

zwei benachbarte $R^{203}$ und $R^{204}$ unabhängig voneinander für einen bivalenten Rest - CH=CH-CH=CH-, wenn m bzw. n für 2 steht.

Ganz besonders stehen in Dihydrophenazinen der Formel (CC)

$R^{201}$ für Phenyl,

$R^{202}$ für Ethyl, Propyl, Butyl, Phenylethyl oder Phenylpropyl,

$R^{203}$ und $R^{204}$ für Wasserstoff und

m und n für 1.

Ein weiterer Gegenstand der Erfindung sind Dihydrophenazine der Formel (CCII),
worin

$R^{202}$ für Alkyl, Cycloalkyl, Alkenyl, Aralkyl oder Aryl, insbesondere für $C_1$- bis $C_{12}$-Alkyl, $C_3$- bis $C_7$-Cycloalkyl, $C_2$- bis $C_{12}$-Alkenyl, $C_7$- bis $C_{16}$-Aralkyl oder $C_6$- bis $C_{10}$-Aryl steht,

B für eine bivalente Brücke, insbesondere für $-(CH_2)_p-$, $-CH_2-(O-CH_2)q-O-CH_2-$ oder $-(CH_2)_r-C_6H_4-(CH_2)_s-$ steht, wobei die $CH_2$-Gruppen durch Methyl substituiert sein können,

$R^{203}$ und $R^{104}$ unabhängig voneinander für Wasserstoff, Halogen, $C_1$- bis $C_4$-Alkyl, $C_1$- bis $C_4$-Alkoxy, Cyano oder $C_6$- bis $C_{10}$-Aryl stehen,

m und n unabhängig voneinander für eine ganze Zahl von 1 bis 4 stehen oder

zwei benachbarte $R^{203}$ und $R^{204}$ unabhängig voneinander für einen bivalenten Rest -CH=CH-CH=CH- stehen, wenn m bzw. n für ≥ 2 steht,

p für eine ganze Zahl von 2 bis 20 steht und

q, r und s unabhängig voneinander für eine ganze Zahl von 0 bis 10 stehen.

Insbesondere stehen in Dihydrophenazinen der Formel (CCII)

$R^{202}$ für gegenenfalls verzweigtes $C_2$- bis $C_8$-Alkyl, Cyclopentyl, Cyclohexyl, Benzyl, Phenethyl, Phenylpropyl oder Phenyl, wobei diese Reste gegebenenfalls bis zu drei Methyl-, Methoxy-, Chlor-, Brom- oder Cyano-Reste tragen können,

B für $-(CH_2)_p-$,

$R^{203}$ und $R^{204}$ unabhängig voneinander für Wasserstoff, Chlor, Brom, Methyl, Ethyl, Propyl, Butyl, Methoxy, Ethoxy, Cyano oder Phenyl,

m und n unabhängig voneinander für eine ganze Zahl von 1 bis 2 oder

zwei benachbarte $R^{203}$ und $R^{204}$ unabhängig voneinander für einen bivalenten Rest -CH=CH-CH=CH-, wenn m bzw. n für 2 steht, und

p für eine ganze Zahl von 2 bis 10.

Ganz besonders stehen in Dihydrophenazinen der Formel (CCII)

$R^{202}$ für Methyl, Ethyl, Propyl, Butyl, Phenethyl, Phenylpropyl oder Phenyl,

B für $-(CH_2)_p-$,

$R^{203}$ und $R^{204}$ für Wasserstoff,

m und n für 1,

p für eine ganze Zahl von 3 bis 5,

In ganz besonderem Maße stehen in Dihydrophenazinen der Formel (CCII)

$R^{202}$ für Phenyl,

B für $-(CH_2)_p-$,

$R^{203}$ und $R^{204}$ für Wasserstoff,

m und n für 1,

p für 3 oder 4.

## Beispiele

### Beispiel 1

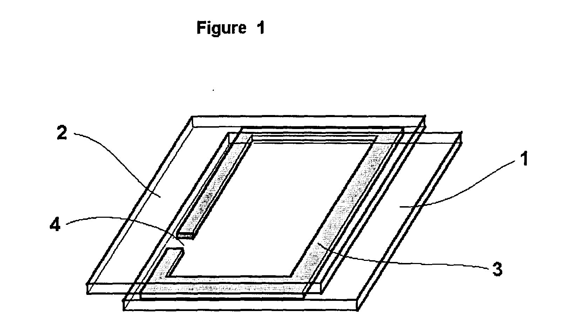

Gemäß Fig. 1 wurde eine Zelle aufgebaut. Es wurden hierzu zwei Glasplatten 1 und 2 benutzt, die auf einer Fläche mit ITO beschichtet sind.

Eine Mischung aus 97 % photohärtendem Epoxikleber DELO-Katiobond® 4594 (DELO Industrieklebstoffe, Landsberg) und 3 % Glaskugeln mit 200 µm Durchmesser wurde ringförmig (3, siehe Fig. 1) auf die mit ITO-beschichtete Seite der Glasplatte 1 so aufgetragen, dass eine 2 mm breite Öffnung (4, siehe Fig. 1) ausgespart wurde. Nun

wurde die Glasplatte 2 so auf die Kleberaupe gelegt, dass die ITO-Schichten der beiden Platten 1 und 2 einander zugewandt waren und eine Geometrie entstand, wie in Fig. 1 gezeigt. Die Aushärtung des Klebers erfolgte durch 10-minütiges Belichten mit Tageslicht in der Nähe eines Fensters und anschließend für 20 min bei 105 °C ohne Belichtung.

Eine Schale wurde unter Stickstoffatmosphäre mit einer Lösung gefüllt, die 0,02 molar an den elektrochromen Verbindungen der Formeln

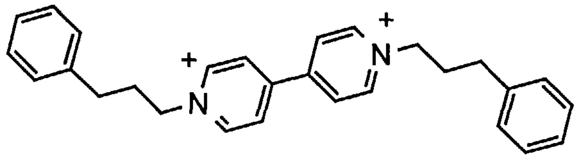

(CCC) und

$2\ BF_4^-$

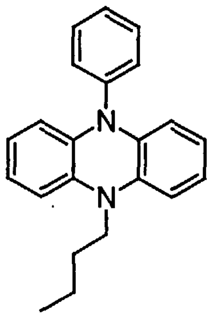

(CCCI)

und jeweils 0,1 molar an den UV-Absorbern der Formeln

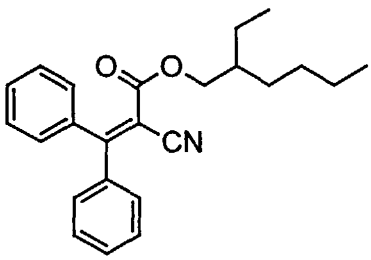

(CCCX)

und

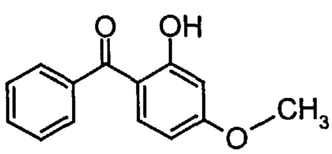

(CCCXI)

in wasserfreiem, sauerstofffreiem Propylencarbonat war.

Dann wurde die Zelle unter Stickstoffatmosphäre senkrecht so in die Schale gestellt, dass die Öffnung 4 sich unterhalb des Flüssigkeitsspiegels befand. Die Schale mit der Zelle wurde in einen Exsiccator gestellt. Dieser wurde auf 0,05 mbar evakuiert und anschließend vorsichtig mit Stickstoff belüftet. Während der Belüftung stieg die elektrochrome Lösung durch die Öffnung 4 in die Zelle hinein und füllte bis auf eine kleine Blase das gesamte Volumen aus. Die Zelle wurde aus der Lösung entnommen, unter Stickstoffatmoshäre an der Öffnung 4 gereinigt, indem sie mit einem Papiertuch abgeputzt wurde, und mit dem photochemisch härtbaren Acrylatkleber DELO-Photobond® 4497 (DELO Industrieklebstoffe, Landsberg) verschlossen. Anschließend wurde 1 min unter Stickstoffatmosphäre mit der Lampe DELOLUX® 03 (DELO Industrieklebstoffe, Landsberg) die sich in einem Abstand von 8 cm zur Öffnung 4 befand, belichtet und bei Raumtemperatur über Nacht unter Stickstoffatmosphäre ausgehärtet.

Durch Anlegen einer Spannung von 0,9 V an die beiden Platten 1 und 2 färbte sich die Zelle rasch tief grünlich blau. Durch Abschalten der Spannung und Kurzschließen der Kontakte verschwand die Färbung wieder rasch.

Ganz analog wurden die folgenden elektrochromen Verbindungen der Beispiele 2 bis 12 eingesetzt:

| Bsp. | $OX_2$ | $RED_1$ |
|---|---|---|
| 2 | 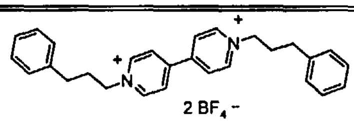 $2\ BF_4^-$ | 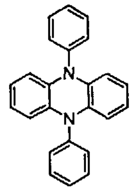 |
| 3 | 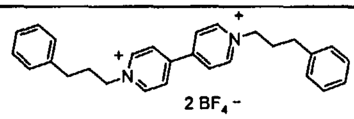 $2\ BF_4^-$ | 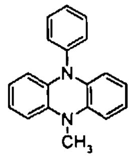 |
| 4 | 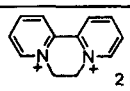 $2\ BF_4^-$ | 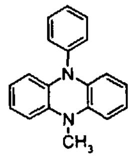 |
| 5 | 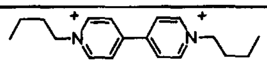 $2\ BF_4^-$ | 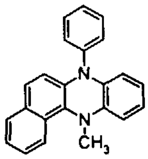 |

| Bsp. | $OX_2$ | $RED_1$ |
|---|---|---|
| 6 | 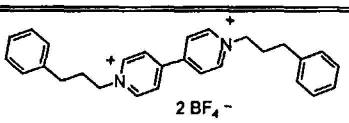 | 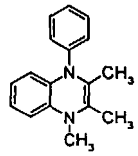 |
| 7 | 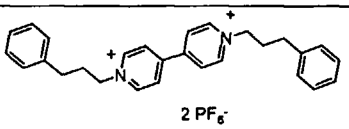 | 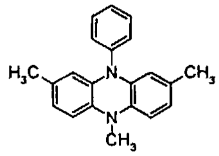 |
| 8 | 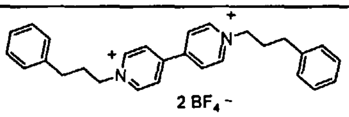 | 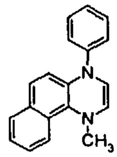 |
| 9 | 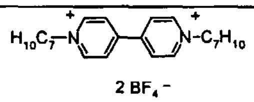 | 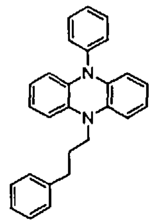 |
| 10 | 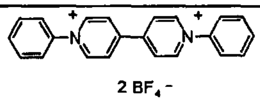 | 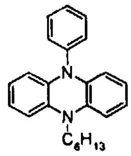 |
| 11 | 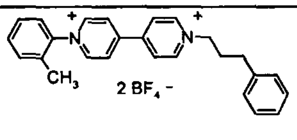 | 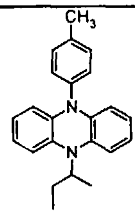 |
| 12 | 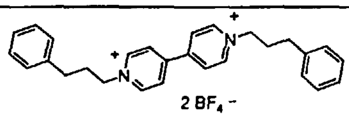 | 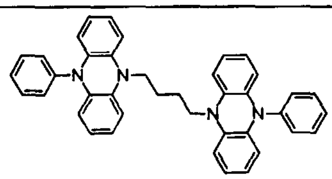 |

## Patentansprüche

1. Elektrochrome Vorrichtung, enthaltend ein Paar Glas- oder Kunststoffplatten oder Kunststoffolien, von denen mindestens eine Platte oder Folie, vorzugsweise beide Platten oder Folien auf jeweils einer Seite mit einer elektrisch leitfähigen Beschichtung versehen sind, von denen wenigstens eine Platte oder Folie und ihre leitfähige Beschichtung transparent sind, von denen die andere verspiegelt sein kann und von denen wenigstens bei einer der beiden Platten oder Folien die elektrisch leitfähige Schicht in getrennte, einzeln kontaktierte Flächensegmente aufgeteilt sein kann, wobei die Platten oder Folien über einen Dichtungsring auf den Seiten ihrer leitfähigen Beschichtung zusammengefügt sind, und das Volumen, gebildet aus den beiden Platten oder Folien und dem Dichtungsring, mit einem elektrochromen Medium gefüllt ist, das ein Paar elektrochromer Substanzen $OX_2$ und $RED_1$ enthält, dadurch gekennzeichnet, dass $RED_1$ einer der Formeln

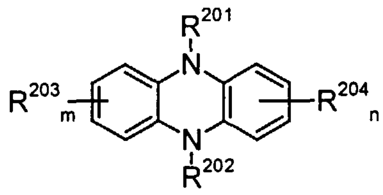

(CC),

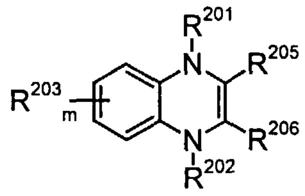

(CCI),

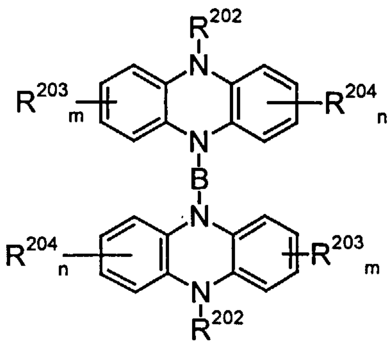

(CCII) oder

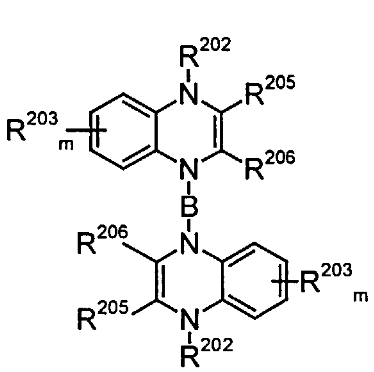

(CCIII)

entspricht,
worin

$R^{201}$ für Aryl steht,

$R^{202}$ für Alkyl, Cycloalkyl, Alkenyl, Aralkyl oder Aryl steht,

B für eine bivalente Brücke steht,

$R^{203}$ bis $R^{206}$ unabhängig voneinander für Wasserstoff, Halogen, Alkyl, Alkoxy, Cyano oder Aryl stehen,

m und n unabhängig voneinander für eine ganze Zahl von 1 bis 4 stehen oder

zwei benachbarte $R^{203}$ und $R^{204}$ unabhängig voneinander für einen bivalenten Rest -CH=CH-CH=CH- stehen, wenn m bzw. n für ≥ 2 steht.

**2.** Elektrochrome Vorrichtung gemäß Anspruch 1, dadurch gekennzeichnet, dass $RED_1$ einer der Formeln (CC), (CCI), (CCII) oder (CCIII) entspricht,
worin

$R^{201}$ für $C_6$- bis $C_{10}$-Aryl steht,

$R^{202}$ für $C_1$- bis $C_{12}$-Alkyl, $C_3$- bis $C_7$-Cycloalkyl, $C_2$- bis $C_{12}$-Alkenyl, $C_7$-bis $C_{16}$-Aralkyl oder $C_6$- bis $C_{10}$-Aryl steht,

B für $-(CH_2)_p-$, $-(CH_2)-(O-CH_2)_q-O-CH_2-$oder $-(CH_2)_r-C_6H_4-(CH_2)_s-$ steht, wobei die $CH_2$-Gruppen durch Methyl substituiert sein können,

$R^{203}$ bis $R^{206}$ unabhängig voneinander für Wasserstoff, Halogen, $C_1$- bis $C_4$-Alkyl, $C_1$- bis $C_4$-Alkoxy, Cyano oder $C_6$- bis $C_{10}$-Aryl stehen,

m und n unabhängig voneinander für eine ganze Zahl von 1 bis 4 stehen oder

zwei benachbarte $R^{203}$ und $R^{204}$ unabhängig voneinander für einen bivalenten Rest -CH=CH-CH=CH- stehen, wenn m bzw. n für ≥ 2 steht,

p für eine ganze Zahl von 2 bis 20 steht und

q, r und s unabhängig voneinander für eine ganze Zahl von 0 bis 10 stehen.

**3.** Elektrochrome Vorrichtung gemäß Anspruch 1, dadurch gekennzeichnet, dass $RED_1$ einer der Formeln (CC), (CCI), (CCII) oder (CCIII) entspricht,
worin

$R^{201}$ für Phenyl steht, das gegebenenfalls bis zu drei Methyl-, Methoxy-, Chlor-, Brom- oder Cyano-Reste tragen kann,

$R^{202}$ für gegebenenfalls verzweigtes $C_1$- bis $C_8$-Alkyl, Cyclopentyl, Cyclohexyl, Benzyl, Phenethyl, Phenylpropyl oder Phenyl steht, wobei diese Reste gegebenenfalls bis zu drei Methyl-, Methoxy-, Chlor-, Brom- oder Cyano-Reste tragen können,

B für $-(CH_2)_p-$ steht,

$R^{203}$ bis $R^{206}$ unabhängig voneinander für Wasserstoff, Chlor, Brom, Methyl, Ethyl, Propyl, Butyl, Methoxy, Ethoxy, Cyano oder Phenyl stehen,

m und n unabhängig voneinander für eine ganze Zahl von 1 bis 2 stehen oder

zwei benachbarte $R^{203}$ und $R^{204}$ unabhängig voneinander für einen bivalenten Rest -CH=CH-CH=CH- stehen, wenn m bzw. n für 2 steht und

p für eine ganze Zahl von 2 bis 10 steht.

**4.** Elektrochrome Vorrichtung gemäß Anspruch 1, dadurch gekennzeichnet, dass $RED_1$ der Formel (CC) entspricht, worin

$R^{201}$ für Phenyl steht,

$R^{202}$ für Methyl, Ethyl, Propyl, Butyl, Phenylpropyl oder Phenyl steht,

$R^{203}$ und $R^{204}$ für Wasserstoff stehen und

m und n jeweils 1 bedeuten.

**5.** Elektrochrome Vorrichtung gemäß Anspruch 4, worin

$R^{201}$ und $R^{202}$ für Phenyl stehen.

**6.** Elektrochrome Vorrichtung gemäß Anspruch 1, dadurch gekennzeichnet, dass $RED_1$ der Formel (CCII) entspricht, worin

$R^{202}$ für Phenyl steht,

B für $-(CH_2)_p-$ steht,

$R^{203}$ und $R^{204}$ für Wasserstoff stehen,

m und n 1 bedeuten und

p für eine ganze Zahl von 2 bis 6 steht.

**7.** Elektrochrome Vorrichtung gemäß Anspruch 6, worin

$R^{202}$ für Phenyl steht und
p für 4 steht.

**8.** Elektrochrome Vorrichtung gemäß Anspruch 1, dadurch gekennzeichnet, dass sie als $OX_2$ eine Verbindung ausgewählt aus den Formeln

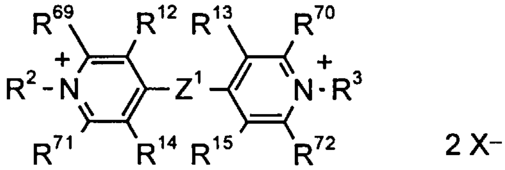

(I),

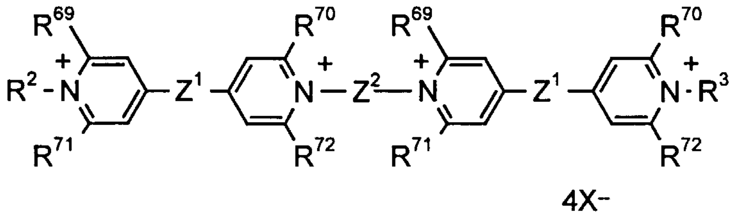

(II),

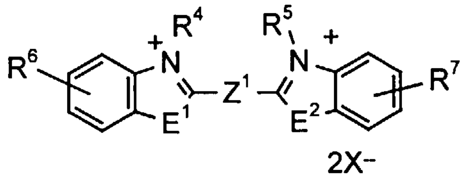

(III),

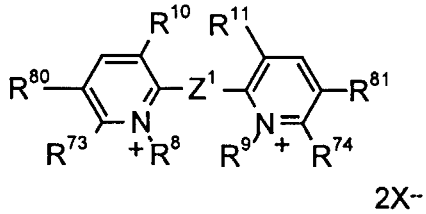

(IV),

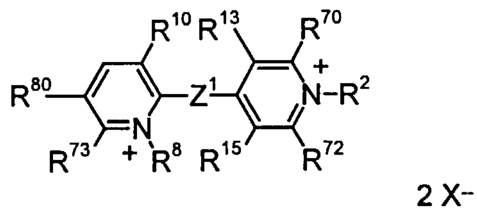

(V),

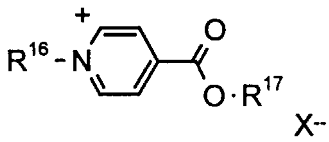

(VI),

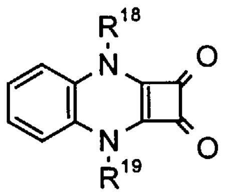

(VII),

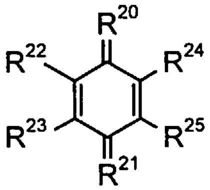

(VIII),

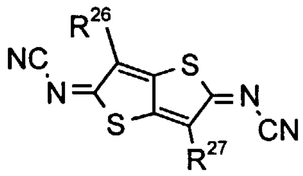

(IX),

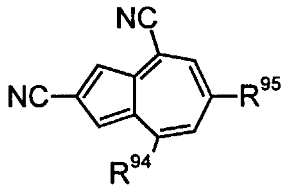

(X),

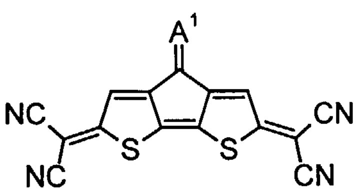

(XI),

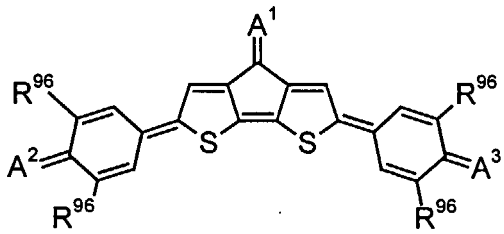

(XII),

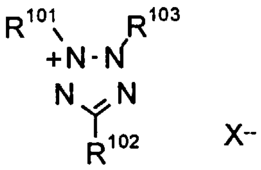

(CI),

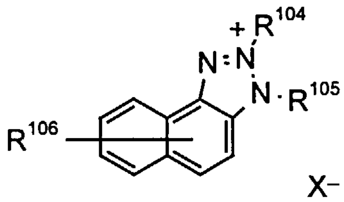

(CII),

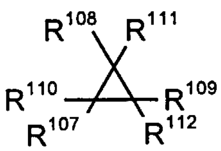

(CIII),

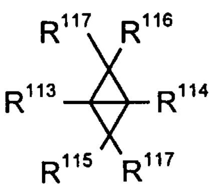

(CIV),

worin

$R^2$ bis $R^5$, $R^8$, $R^9$, $R^{16}$ bis $R^{19}$ unabhängig voneinander $C_1$- bis $C_{18}$-Alkyl, $C_2$-bis $C_{12}$-Alkenyl, $C_4$- bis $C_7$-Cycloalkyl, $C_7$,- bis $C_{15}$-Aralkyl oder $C_6$-bis $C_{10}$-Aryl bedeuten oder

$R^4$; $R^5$ bzw. $R^8$; $R^9$ gemeinsam eine $-(CH_2)_2-$ oder $-(CH_2)_3$-Brücke bilden können,

$R^6$, $R^7$ und $R^{22}$ bis $R^{25}$ unabhängig voneinander Wasserstoff, $C_1$- bis $C_4$-Alkyl, $C_1$- bis $C_4$-Alkoxy, Halogen, Cyan, Nitro oder $C_1$- bis $C_4$-Alkoxycarbonyl bedeuten oder

$R^{22}$; $R^{23}$ und/oder $R^{24}$; $R^{25}$ eine -CH=CH-CH=CH-Brücke bilden können,

$R^{10}$; $R^{11}$, $R^{10}$; $R^{13}$, $R^{12}$; $R^{13}$ und $R^{14}$; $R^{15}$ unabhängig voneinander Wasserstoff oder paarweise eine $-(CH_2)_2-$, $-(CH_2)_3-$ oder -CH=CH-Brücke bedeuten,

$R^{20}$ und $R^{21}$ unabhängig voneinander O, N-CN, $C(CN)_2$ oder N-$C_6$- bis $C_{10}$-Aryl bedeuten,

$R^{26}$ und $R^{27}$ Wasserstoff, $C_1$- bis $C_4$-Alkyl, $C_1$- bis $C_4$-Alkoxy, Halogen, Cyan, Nitro, $C_1$- bis $C_4$-Alkoxycarbonyl oder $C_6$- bis $C_{10}$-Aryl bedeuten,

$R^{69}$ bis $R^{74}$, $R^{80}$ und $R^{81}$ unabhängig voneinander Wasserstoff oder $C_1$- bis $C_6$-Alkyl bedeuten oder

$R^{69}$; $R^{12}$, $R^{70}$; $R^{13}$, $R^{73}$; $R^{80}$ und/oder $R^{74}$; $R^{81}$ gemeinsam eine -CH=CH-CH=CH-Brücke bilden,

$E^1$ und $E^2$ unabhängig voneinander O, S, $NR^1$ oder $C(CH_3)_2$ bedeuten oder

$E^1$ und $E^2$ gemeinsam eine $-N-(CH_2)_2-N-$Brücke bilden,

$R^1$ $C_1$- bis $C_{18}$-Alkyl, $C_2$- bis $C_{12}$-Alkenyl, $C_4$- bis $C_7$-Cycloalkyl, $C_7$- bis $C_{15}$-Aralkyl, $C_6$- bis $C_{10}$-Aryl bedeutet,

$Z^1$ eine direkte Bindung, -CH=CH-, $-C(CH_3)=CH-$, -C(CN)=CH-, -CCl=CCl-, -C(OH)=CH-, -CCl=CH-, -C≡C-, -CH=N-N=CH-, $-C(CH_3)=N-N=C(CH_3)-$ oder -CCl=N-N=CCl- bedeutet,

$Z^2$ $-(CH_2)_r-$ oder $-CH_2-C_6H_4-CH_2-$ bedeutet,

r eine ganze Zahl von 1 bis 10 bedeutet,

$R^{94}$ und $R^{95}$ unabhängig voneinander Wasserstoff oder Cyano bedeuten,

$R^{101}$ bis $R^{105}$ unabhängig voneinander $C_6$- bis $C_{10}$-Aryl oder einen ggf. benzanellierten aromatischen oder quasiaromatischen fünf- oder sechsgliedrigen heterocyclischen Ring bedeuten,

$R^{107}$, $R^{109}$, $R^{113}$ und $R^{114}$ unabhängig voneinander einen Rest der Formeln (CV) bis (CVII)

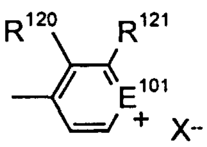

(CV),

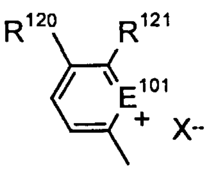

(CVI),

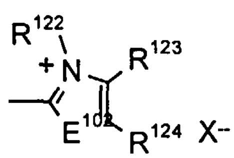

(CVII)

bedeuten,

$R^{108}$, $R^{115}$ und $R^{116}$ unabhängig voneinander $C_6$- bis $C_{10}$-Aryl oder einen Rest der Formel (CV) bedeuten,

$R^{110}$ bis $R^{112}$, $R^{117}$ und $R^{118}$ unabhängig voneinander Wasserstoff, $C_1$- bis $C_4$-Alkyl, Halogen oder Cyano

bedeuten,

$E^{101}$ und $E^{102}$ unabhängig voneinander O, S oder N-$R^{119}$ bedeuten,

$R^{119}$ und $R^{122}$ unabhängig voneinander $C_1$- bis $C_{18}$-Alkyl, $C_2$- bis $C_8$-Alkenyl, $C_4$- bis $C_7$-Cycloalkyl, $C_7$- bis $C_{15}$-Aralkyl oder $C_6$- bis $C_{10}$-Aryl bedeuten,

$R^{106}$, $R^{120}$, $R^{121}$, $R^{123}$ und $R^{124}$ unabhängig voneinander Wasserstoff, $C_1$- bis $C_4$-Alkyl, $C_1$- bis $C_4$-Alkoxy, Halogen, Cyano, Nitro oder $C_1$- bis $C_4$-Alkoxycarbonyl bedeuten oder

$R^{120}$, $R^{121}$ bzw. $R^{123}$, $R^{124}$ gemeinsam eine -CH=CH-CH=CH-Brücke bilden,

$A^1$, $A^2$ und $A^3$ unabhängig voneinander O oder $C(CN)_2$ bedeuten,

$R^{96}$ Wasserstoff, Phenyl oder tert.-Butyl bedeutet und

$X^-$ ein unter den Bedingungen redox-inertes Anion bedeutet,

enthält.

**9.** Verwendung der elektrochromen Vorrichtung gemäß Anspruch 1 als Fenster oder Trennscheibe oder Sichtschutzscheibe oder Verglasung oder Dachverglasung oder Lichtfilter oder Spiegel oder Anzeigevorrichtung.

**10.** Dihydrophenazine der Formel (CC),

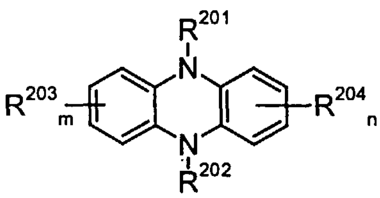

(CC),

worin

$R^{201}$ für $C_6$- bis $C_{10}$-Aryl steht,

$R^{202}$ für $C_2$- bis $C_{12}$-Alkyl, $C_3$- bis $C_7$-Cycloalkyl, $C_2$- bis $C_{12}$-Alkenyl oder $C_7$- bis $C_{16}$-Aralkyl steht,

$R^{203}$ und $R^{204}$ unabhängig voneinander für Wasserstoff, Halogen, $C_1$- bis $C_4$-Alkyl, $C_1$- bis $C_4$-Alkoxy, Cyano oder $C_6$- bis $C_{10}$-Aryl stehen,

m und n unabhängig voneinander für eine ganze Zahl von 1 bis 4 stehen oder

zwei benachbarte $R^{203}$ und $R^{204}$ unabhängig voneinander für einen bivalenten Rest -CH=CH-CH=CH- stehen, wenn m bzw. n für $\geq 2$ steht.

**11.** Dihydrophenazine der Formel (CCII),

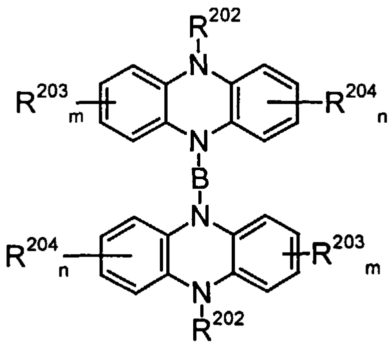

(CCII)

worin

$R^{202}$ für $C_1$- bis $C_{12}$-Alkyl, $C_3$- bis $C_7$-Cycloalkyl, $C_2$- bis $C_{12}$-Alkenyl, $C_7$- bis $C_{16}$-Aralkyl oder $C_6$- bis $C_{10}$-Aryl steht,

B für $-(CH_2)_p-$, $CH_2$, $-(O-CH_2)q-O-CH_2$ oder $-(CH_2)_r-C_6H_4-(CH_2)_s-$steht, wobei die $CH_2$-Gruppen durch Methyl substituiert sein können,

$R^{203}$ und $R^{204}$ unabhängig voneinander für Wasserstoff, Halogen, $C_1$- bis $C_4$-Alkyl, $C_1$- bis $C_4$-Alkoxy, Cyano oder $C_6$- bis $C_{10}$-Aryl stehen,

m und n unabhängig voneinander für eine ganze Zahl von 1 bis 4 stehen oder

zwei benachbarte $R^{203}$ und $R^{204}$ unabhängig voneinander für einen bivalenten Rest -CH=CH-CH=CH- stehen, wenn m bzw. n für ≥ 2 steht,

p für eine ganze Zahl von 2 bis 20 steht und

q, r und s unabhängig voneinander für eine ganze Zahl von 0 bis 10 stehen.

**12.** Elektrochromes Medium, das ein Paar elektrochromer Substanzen $OX_2$ und $RED_1$ enthält, dadurch gekennzeichnet, dass $RED_1$ einer der Formeln

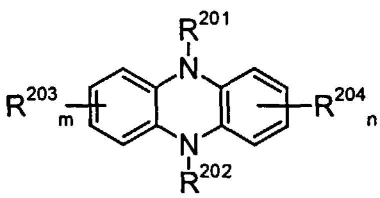

(CC),

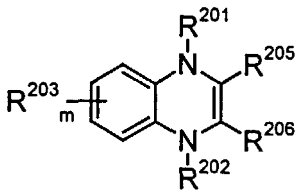

(CCI),

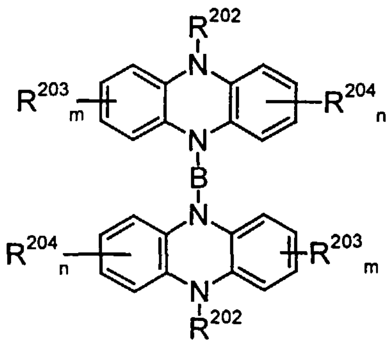

(CCII) oder

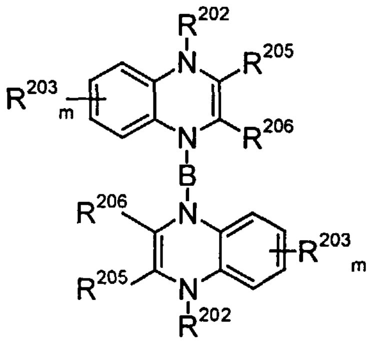

(CCIII)

entspricht,
worin

$R^{201}$ für Aryl steht,

$R^{202}$ für Alkyl, Cycloalkyl, Alkenyl, Aralkyl oder Aryl steht,

B für eine bivalente Brücke steht,

$R^{203}$ bis $R^{206}$ unabhängig voneinander für Wasserstoff, Halogen, Alkyl, Alkoxy, Cyano oder Aryl stehen,

m und n unabhängig voneinander für eine ganze Zahl von 1 bis 4 stehen oder

zwei benachbarte $R^{203}$ und $R^{204}$ unabhängig voneinander für einen bivalenten Rest -CH=CH-CH=CH- stehen, wenn in bzw. n für $\geq 2$ steht.

Figure 1